# EUROPÄISCHE PATENTSCHRIFT

(11) **EP 0 381 798 B1**
(45) Veröffentlichungstag und Bekanntmachung des Hinweises auf die Patenterteilung: **27.07.1994**
(21) Anmeldenummer: 89102347.5
(22) Anmeldetag: 10.02.1989
(51) Int. Cl.: A61N 1/365

(54) **An die körperliche Aktivität angepasstes medizinisches Stimulationsgerät**
Medical stimulation apparatus adapted to the corporal activity
Appareil médical de stimulation adapté à l'activité corporelle

(43) Veröffentlichungstag der Anmeldung: 16.08.1990
(73) Patentinhaber: Pacesetter AB, 171 95 Solna (SE)
(72) Erfinder: Lekholm Anders, Dr., S-161 39 Bromma (SE)
(74) Vertreter: Lettström, Richard Wilhelm

(56) Entgegenhaltungen:
- EP-A- 0 222 681
- EP-A- 0 229 886

## Beschreibung

Medizinisches Gerät zur Stimulation eines physiologischen Vorganges eines Lebewesens mit sich selbsttätig an die körperliche Aktivität des Lebewesens anpassender Stimulationsintensität sowie Verfahren zur Anpassung der Stimulationsintensität an die körperliche Aktivität eines Lebewesens.

Die Erfindung betrifft ein in den Körper eines Lebewesens implantierbares medizinisches Gerät zur Stimulation eines physiologischen Vorganges des Lebewesens, welches Gerät Mittel zur Stimulation des physiologischen Vorgangs mit einstellbarer Stimulationsintensität und selbsttätige Stellmittel zur Anpassung der Stimulationsintensität an die körperliche Aktivität des Lebewesens aufweist, die die Stimulationsintensität anhand der Signale zweier Temperatursensoren einstellen, von denen das eine der Temperatur im Bereich des Körperkerns und das andere der Temperatur im Bereich der Körperperipherie des Lebewesens entspricht. Die Erfindung betrifft ausserdem ein Verfahren zur Anpassung der einstellbaren Stimulationsintensität eines medizinischen Gerätes zur Stimulation eines physiologischen Vorganges eines Lebewesens an die körperliche Aktivität des Lebewesens, wobei die Stimulationsintensität anhand der Temperatur im Bereich des Körperkernes und der Temperatur im Bereich der Körperperipherie des Lebewesens eingestellt wird. Der Begriff Stimulationsintensität soll hier umfassend verstanden werden, d.h., dass sowohl die Dauer als auch die Häufigkeit, die Folgefrequenz, die Amplitude etc., mit der die Mittel zur Stimulation tätig werden, einzeln und/oder in Kombination als Mass für die Stimulationsintensität verstanden werden sollen.

Ein Gerät und ein Verfahren der eingangs genannten Art sind aus der EP-A-0 222 681 bekannt. Diese Druckschrift betrifft einen Herzschrittmacher, bei dem die Mittel zur Stimulation eines physiologischen Vorganges die Herztätigkeit des Lebewesens stimulieren und die Stellmittel zum Einstellen der Stimulationsintensität die Stimulationsfrequenz, mit der die Mittel zur Stimulation der Herztätigkeit das Herz des Lebewesens im Bedarfsfalle stimulieren, an die körperliche Aktivität des Lebewesens anpassen. Dabei erfolgt die Anpassung der Stimulationsfrequenz bei beginnender körperlicher Aktivität anhand der Differenz zwischen der im Bereich des Körperkernes und der im Bereich der Körperperipherie des Lebewesens vorliegenden Temperatur. Bei langanhaltender körperlicher Aktivität erfolgt die Anpassung der Stimulationsfrequenz auf andere Weise, beispielsweise anhand der im Bereich des Körperkernes des Lebewesens vorliegenden Temperatur.

Da eine Differenz zwischen der Temperatur im Bereich des Körperkernes und der Temperatur im Bereich der Körperperipherie von bestimmten Ausnahmefällen abgesehen praktisch immer vorhanden ist und auch Änderungen dieser Differenz nicht unbedingt im Zusammenhang mit einer Änderung der körperlichen Aktivität des Lebewesens stehen müssen, besteht im Falle des bekannten Herzschrittmachers die Gefahr, dass die Stimulationsfrequenz geändert wird, ohne dass eine entsprechende Änderung körperlicher Aktivität vorliegt. Dies muss zwar nicht unbedingt eine Gefahr für das den Herzschrittmacher tragende Lebewesen darstellen, bereitet diesem aber zumindest Unbehagen. Auch muss eine Änderung der im Bereich des Körperkernes vorhandenen Temperatur (Bluttemperatur), die im Falle des bekannten Herzschrittmachers zur Anpassung der Stimulationsfrequenz an längerfristige körperliche Aktivitäten herangezogen wird, nicht zwangsläufig auf einer geänderten körperlichen Aktivität des Lebewesens beruhen, so dass auch hier die Gefahr von Fehlanpassungen der Stimulationsfrequenz besteht.

Der Erfindung liegt daher die Aufgabe zugrunde, ein Gerät und ein Verfahren der eingangs genannten Art so auszubilden, dass zuverlässig unterschieden werden kann, ob eine Änderung der Körpertemperatur auf einer Änderung der körperlichen Aktivität beruht oder andere Ursachen hat.

Der das Gerät betreffende Teile der Aufgabe wird nach der Erfindung dadurch gelöst, dass die Stellmittel die Stimulationsintensität unter Ermittlung der zeitlichen Reihenfolge einstellen, in der Änderungen der zeitlichen Verläufe beider Temperaturen auftreten. Liegen stationäre Bedingungen vor, wird die Temperatur im Bereich des Körperkernes gewöhnlich über der im Bereich der Körperperipherie liegen. Nimmt ausgehend von einem stationären Zustand die körperliche Aktivität des Lebewesens zu, wächst sowohl die Temperatur im Bereich des Körperkernes als auch die Temperatur im Bereich der Körperperipherie an. Da jedoch das Körpergewebe eine hohe spezifische Wärme und eine geringe Wärmeleitfähigkeit aufweist, wird gewöhnlich eine durch zunehmende körperliche Aktivität des Lebewesens bedingte Temperaturerhöhung im Bereich des Körperkernes infolge der in den Muskeln anfallenden Verlustwärme früher auftreten als der entsprechende Anstieg der Temperatur im Bereich der Körperperipherie. Umgekehrt wird für den Fall, dass sich das Lebewesen z.B. von einem kalten in einen warmen Raum begibt, zunächst eine Erhöhung der Temperatur im Bereich der Körperperipherie auftreten und eine entsprechende Erhöhung der Temperatur im Bereich des Körperkernes mit zeitlicher Verzögerung erfolgen. Die zeitliche Reihenfolge, in der Änderungen des zeitlichen Verlaufs der im Bereich des Körperkernes bzw. im Bereich der Körperperipherie gemessenen Temperatur erfolgen, lässt also die Flussrichtung der Wärme erkennen und ist somit ein Indikator dafür, ob eine Änderung der Körpertemperatur auf eine innerhalb (körperliche Aktivität) oder ausserhalb (z.B. hohe Raumtemperatur) des Körpers des Lebewesens befindliche Wärmequelle zurückgeht. Das erfindungsgemässe Gerät vermag also festzustellen, ob eine Änderung der Körpertemperatur auf einer Änderung der körperlichen Aktivität des Lebewesens beruht oder andere Ursachen hat, so dass die Stellmittel tatsächlich nur dann zur Einstellung der Stimulationsintensität auf die Mittel zur Stimulation einwirken, wenn hierfür ein Anlass besteht.

Eine nochmals differenziertere Reaktion der Stellmittel ist möglich, wenn die Stellmittel die Stimulationsintensität ausserdem unter Berücksichtigung von wenigstens dem Vorzeichen der Steigung des zeitlichen Verlaufs zumindest einer der Temperaturen einstellen.

Der ein Gerät betreffende Teil der Aufgabe wird gemäss einem weiteren Ausführungsbeispiel der Erfindung dadurch gelöst, dass die Stellmittel die Stimulationsintensität unter Berücksichtigung des zeitlichen Verlaufs zumindest einer der Temperaturen wenigstens dem Vorzeichen von dessen Steigung nach und unter Berücksichtigung des zeitlichen Verlaufs der Differenz der beiden Temperaturen wenigstens hinsichtlich des Über- oder Unterschreitens eines Normalwertes der Differenz einstellen. Auch in diesem Falle vermag das Gerät zu unterscheiden, ob eine Änderung der Körpertemperatur auf einer Änderung der körperlichen Aktivität beruht oder andere Ursachen hat, da das Vorzeichen der Steigung des zeitlichen Verlaufs wenigstens einer der Temperaturen, vorzugsweise der Temperatur im Bereich des Körperkernes, in Verbindung mit dem Über- oder Unterschreiten eines Normalwertes der Differenz der beiden Temperaturen eine Aussage darüber erlaubt, ob ein Wärmetransport vom Inneren des Körpers des Lebewesens nach aussen oder umgekehrt erfolgt.

Im Falle beider Lösungsprinzipien wird beim Auftreten eines stationären Zustandes der Temperaturen jeweils die zuletzt aufgetretene Änderung des jeweils betrachteten Parameters berücksichtigt. Bei beiden Lösungsprinzipen wird als Mass für die Intensität der körperlichen Aktivität des Lebewesens vorzugsweise eine der beiden Temperaturen, insbesondere die im Bereich des Körperkerns vorliegende Temperatur, berücksichtigt. Der wesentliche Vorteil beider Lösungsprinzipien liegt darin, dass im Falle einer Änderung der Körpertemperatur jeweils die Richtung des Wärmetransportes ermittelt wird, wodurch das Gerät festzustellen vermag, ob eine Änderung, insbesondere eine Erhöhung, der Körpertemperatur des Lebewesens auf einer Änderung, insbesondere einer Erhöhung der körperlichen Aktivität oder einer anderen Ursache beruht.

Eine weiter differenzierte Beurteilung der Ursachen von Änderungen der Körpertemperatur ist im Falle beider Lösungsprinzipien möglich, wenn die Stellmittel die Stimulationsintensität ausserdem unter Berücksichtigung des Betrages der Steigung des zeitlichen Verlaufs wenigstens einer der Temperaturen einstellen. Ausserdem kann es zweckmässig sein, wenn die Stellmittel die Stimulationsintensität unter Berücksichtigung wenigstens der Vorzeichen der Steigungen der zeitlichen Verläufe beider Temperaturen einstellen.

Eine Variante der Erfindung sieht vor, dass das Gerät als Herzschrittmacher ausgeführt ist, wobei die Mittel zur Stimulation eines physiologischen Vorgangs die Herztätigkeit des Lebewesens stimulieren und die Stellmittel zum Einstellen der Stimulationsintensität die Stimulationsfrequenz, mit der die Mittel zur Stimulation das Herz des Lebewesens im Bedarfsfalle stimulieren, an die körperliche Aktivität des Lebewesens anpassen.

Der ein Verfahren betreffende Teil der Aufgabe wird dadurch gelöst, dass die Stimulationsintensität unter Ermittlung der zeitlichen Reihenfolge eingestellt wird, in der Änderungen der zeitlichen Verläufe beider Temperaturen auftreten, wobei gemäss einer besonders vorteilhaften Variante der Erfindung die Stimulationsintensität ausserdem unter Berücksichtigung wenigstens des Vorzeichens der Steigung des zeitlichen Verlaufs zumindest einer der Temperaturen eingestellt wird.

Ein zweites Ausführungsbeispiel sieht vor, dass die Stimulationsintensität unter Berücksichtigung des zeitlichen Verlaufs zumindest einer der Temperaturen wenigstens dem Vorzeichen der Steigung nach und des zeitlichen Verlaufs der Differenz beider Temperaturen wenigstens hinsichtlich des Über- oder Unterschreitens eines Normalwertes der Differenz eingestellt wird.

Die Vorteile und die Wirkungsweise der erfindungsgemässen Verfahren ergeben sich aus der Erläuterung der erfindungsgemässen Geräte.

Weitere Einzelheiten und Vorteile der Erfindung ergeben sich aus der Beschreibung zweier Ausführungsbeispiele, die anhand der beigefügten Zeichnungen erfolgt. Es zeigen:
- FIG 1: In schematischer Darstellung das Blockschaltbild eines erfindungsgemässen Gerätes,
- FIG 2 bis 4: in Diagrammform für drei verschiedene Situationen die Verläufe der im Bereich des Körperkernes und im Bereich der Körperperipherie vorliegenden Temperaturen und den zeitlichen Verlauf der Differenz dieser Temperaturen,
- FIG 5: in schematischer Darstellung als Blockschaltbild eine weitere Ausführungsform des erfindungsgemässen Gerätes, und
- FIG 6: das Gerät gemäss FIG 5 im implantierten Zustand.

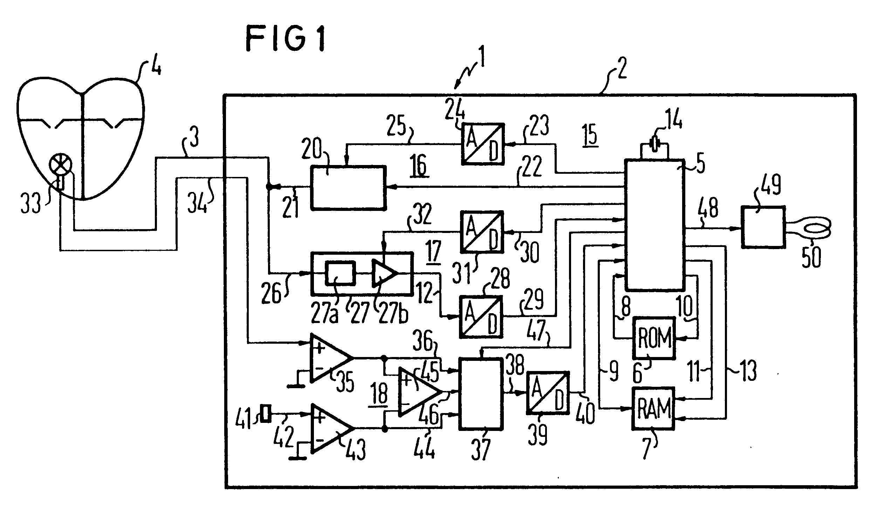

In FIG 1 ist die Erfindung anhand eines insgesamt mit (1) bezeichneten Herzschrittmachers dargestellt. Die Bauteile des Herzschrittmachers (1) sind in einem schematisch angedeuteten hermetisch dichten Gehäuse (2) aufgenommen. Von dem im VVI-Mode arbeitenden Herzschrittmacher (1) führt eine Elektrode (3) zu einem Herz (4) eines Lebewesens und ist dort in das rechte Ventrikel eingepflanzt.

Der Herzschrittmacher (1) umfasst unter anderem einen Mikroprozessor (5), dem ein Nur-Lese-Speicher (ROM) (6) und ein Schreib-Lese-Speicher (RAM) (7) zugeordnet sind, die über Datenleitungen (8,9) und Adressleitungen (10,11) mit dem Mikroprozessor (5) in Verbindung stehen. Zu dem RAM (7) führt von dem Mikroprozessor (5) ausserdem eine Leitung (13), die zum Umschalten des RAM (7) von Schreib-auf Lesebetrieb und umgekehrt dient. In dem ROM (6) ist ein Programm gespeichert, mittels dessen sämtliche Funktionen des Herzschrittmachers (1) gesteuert werden. Wenn also im folgenden die Rede davon ist, dass der Mikroprozessor (5) eine bestimmte Funktion ausführt, so ist darunter zu verstehen, dass der Mikroprozessor (5) unter Ausführung des im ROM (6) gespeicherten Programmes bei Heranziehung von im RAM (7) befindlichen Daten und ihm anderweitig, z.B. über eine Eingangs-Beschaltung, zugeführter Daten zur Ausführung der jeweiligen Funktion tätig wird.

Mit dem Mikroprozessor (5) ist ein Quarz (14) verbunden, der zur Erzeugung der für den Betrieb des Mikroprozessors (5) erforderlichen Taktsignale dient und ausserdem die Zeitreferenz für den Betrieb des Herzschrittmachers (1) darstellt.

Der Mikroprozessor (5) des Herzschrittmachers (1) besitzt eine insgesamt mit (15) bezeichnete Eingangs/Ausgangs-Beschaltung, die mehrere Kanäle (16,17,18) aufweist.

Der Kanal (16) dient dazu, das Herz (4) erforderlichenfalls mit Stimulationsimpulsen zu versorgen. Der Kanal (16) besitzt daher einen Stimulationsimpuls-Generator (20), dessen Ausgangsleitung (21) mit der Elektrode (3) verbunden ist. Der Stimulationsimpuls-Generator (20) ist über eine Leitung (22), die mit einem entsprechenden Ausgang des Mikroprozessors (5) verbunden ist, zur Abgabe eines Stimulationsimpulses aktivierbar. Digitale Daten, die den Energiegehalt der Stimulationsimpulse, z.B. deren Amplitude und/oder Dauer, betreffen, gelangen von dem Mikroprozessor (5) über eine Leitung (23) zu einer Digital/Analog-Schnittstelle (24), die den Stimulationsimpuls-Generator (20) über eine Steuerleitung (25) den digitalen Daten entsprechende analoge Steuersignale zuführt, die den Stimulationsimpuls-Generator (20) derart einstellen, dass er bei Bedarf Stimulationsimpulse des gewünschten Energiegehaltes erzeugt.

Der Kanal (17) besitzt eine über eine Eingangsleitung (26) ebenfalls mit der Elektrode (3) verbundene Signalaufbereitungsschaltung (27), die dazu dient, ein mittels der Elektrode (3) vom Herzen (4) abgenommenes, der Aktivität des Herzens entsprechendes elektrisches Signal zu filtern und zu verstärken. Die Signalaufbereitungsschaltung (27) umfasst daher ein Filter (27a) und einen Verstärker (27b). Vom Ausgang der Signalaufbereitungsschaltung (27) gelangt das aufbereitete Signal über eine Leitung (12) zu einem Analog/Digital-Wandler (28). Von diesem gelangen über eine Leitung (29) digitale Daten zu einem entsprechenden Eingang des Mikroprozessors (5), die dem Verlauf des am Ausgang der Signalaufbereitungsschaltung (27) elektrischen Signales entsprechen, das seinerseits die elektrische Aktivität des Herzens (4) wiederspiegelt. Der Mikroprozessor (5) ist über eine Leitung (30) mit einer Digital/Analog-Schnittstelle (31) verbunden, die ihr von dem Mikroprozessor (5) zugeführte digitale Daten als entsprechende analoge Signale über eine Steuerleitung (32) an die Signalaufbereitungsschaltung (27) weitergibt. Die digitalen Daten bzw. die entsprechenden analogen Signale dienen dazu, z.B. den Verstärkungsfaktor des Verstärkers (27b) einzustellen bzw. den Verstärker (27b) vollständig zu sperren.

Die über die Leitung (29) dem Mikroprozessor (5) zugeführten digitalen Daten analysiert dieser daraufhin, ob in dem der Aktivität des Herzens (4) entsprechenden elektrischen Signal Ereignisse enthalten sind, die dem Auftreten eines natürlichen Herzschlages entsprechen. Detektiert der Mikroprozessor (5) einen natürlichen Herzschlag oder aktiviert er den Stimulationsimpuls-Generator (20) zur Abgabe eines Stimulationsimpulses, beginnt der Mikroprozessor (5) als Zähler zu arbeiten und beginnt eine Anzahl von aus der Schwingung des Quarzes (14) abgeleiteten Taktimpulse abzuzählen, die einem zwischen einer oberen und einer unteren Grenze einstellbaren Zeitintervall entspricht. Das jeweils eingestellte Zeitintervall bestimmt diejenige Stimulationsfrequenz mit der das Herz (4) beim Ausbleiben natürlicher Herzschläge stimuliert wird. Gelangen während eines der gewünschten Herzschlagfrequenz entsprechenden Zeitintervalles über den Kanal (17) keine Daten zu dem Mikroprozessor (5), die dieser als natürlichen Herzschlag detektiert, aktiviert der Mikroprozessor (5) bei Ablauf des Zeitintervalls den Stimulationsimpuls-Generator (20). Im Anschluss an die Abgabe eines Stimulationsimpulses beginnt der Mikroprozessor (5) erneut eine Anzahl von Taktimpulsen abzuzählen, die dem jeweils eingestellten, die Stimulationsfrequenz bestimmenden Zeitintervall entspricht. Detektiert der Mikroprozessor (5) dagegen während des Laufs des jeweils eingestellten, die Stimulationsfrequenz bestimmenden Zeitintervalls einen natürlichen Herzschlag, bricht er den beschriebenen Zählvorgang ab, sofern ein weiteres Zeitintervall, die sogenannte Refraktärzeit, abgelaufen ist, und beginnt den beschriebenen Zählvorgang von neuem.

Die Refraktärzeit, die grundsätzlich kürzer ist als das zwischen beispielsweise 400 und 2000 ms einstellbare, die Stimulationsfrquenz bestimmende Zeitintervall, dauert zwischen etwa 250 und 450 ms (einstellbar). Die Refraktärzeit unterteilt sich in eine absolute Refraktärzeit mit einer festen Dauer von gewöhnlich 125 ms und eine relative Refraktärzeit, auf die der restliche Teil der gesamten jeweils eingestellten Refraktärzeit entfällt. Die Refraktärzeit beginnt jeweils gleichzeitig mit dem die Stimulationsfrequenz bestimmenden Zeitintervall zu laufen und wird von dem Mikroprozessor (5) durch den gleichen Zählvorgang ermittelt, der auch zur Ermittlung des die Stimulationsfrequenz bestimmenden Zeitintervalles dient. Während der absoluten Refraktärzeit ist im Kanal (17) der Verstärker (27b) der Signalaufbereitungsschaltung (27) vollständig gesperrt, was dadurch erreicht wird, dass der Mikroprozessor (5) den Verstärker (27b) über die Leitung (30), die Digital/Analog-Schnittstelle (31) und die Steuerleitung (32) mit einem entsprechenden Steuersignal beaufschlagt. Infolge der vollständigen Sperrung des Verstärkers (27b) ist mittels des Mikroprozessors (5) während der Dauer der absoluten Refraktärzeit keinerlei Detektion möglich. Nach Ablauf der absoluten Refraktärzeit aktiviert der Mikroprozessor (5) den Verstärker (27b) wieder, so dass er in der Lage ist, natürliche Herzschläge zu detektieren. Detektiert der Mikroprozessor (5) während der relativen Refraktärzeit einen natürlichen Herzschlag, bricht er im Gegensatz zu einer Detektion nach Ablauf der Refraktärzeit den Zählvorgang zur Ermittlung des die Stimulationsfrequenz bestimmenden Zeitintervalles nicht ab, sondern führt ihn fort und schliesst ihn mit einer Aktivierung des Stimulationsimpuls-Generators (20) ab. Allerdings setzt der Mikroprozessor (5) nach der Detektion eines natürlichen Herzschlages nochmals die volle Refraktärzeit in Gang. Hierdurch wird erreicht, dass im Falle von Hochfrequenzstörungen, die zu Fehldetektionen führen, unabhängig vom Auftreten natürlicher Herzschläge Stimulationsimpulse mit der durch das jeweils eingestellte Zeitintervall bestimmten Stimulationsfrequenz erzeugt werden. Auch wenn die spontane Herzschlagfrequenz so hoch liegt, dass das Auftreten natürlicher Herzschläge jedesmal innerhalb der relativen Refraktärzeit erfolgt, erfolgt eine Abgabe von Stimulationsimpulsen mit der durch das jeweils eingestellte Zeitintervall bestimmten Stimulationsfrequenz, und zwar bis die spontane Herzschlagfrequenz unter eine Frequenz zurückgefallen ist, deren Periodendauer der jeweils eingestellten Refraktärzeit entspricht. Mittels dieser Funktion ist die Beendigung bestimmter Wiedereintrittstachykardien möglich.

Der Kanal (18) dient dazu, dem Mikroprozessor (5) der körperlichen Aktivität des den Herzschrittmacher (1) tragenden Lebewesens entsprechende Daten zuzuführen, die es dem Mikroprozessor (5) gestatten, das die Stimulationsfrequenz bestimmende Zeitintervall in Anpassung an die körperliche Aktivität des Lebewesens einzustellen. Zu diesem Zweck ist die Elektrode (3) im Bereich ihres in das rechte Ventrikel des Herzens (4) eingepflanzten Endes mit einem Temperatursensor (33) versehen, der sich im Inneren des rechten Ventrikels befindet und somit die Temperatur des venösen Blutes misst. Diese Temperatur stellt infolge des Umstandes, dass die in den Muskeln des Lebewesens anfallende Verlustenergie in beachtlichem Masse durch das venöse Blut abtransportiert wird, einen brauchbaren Indikator für die körperliche Aktivität des Lebewesens dar. Das Signal des Temperatursensors (33) gelangt über eine parallel zu der Elektrode (3) verlaufende Leitung (34), die in das Gehäuse (2) des Herzschrittmachers (1) geführt ist, zu einem Operationsverstärker (35), der das Signal verstärkt. Vom Ausgang des Operationsverstärkers (35) gelangt das Signal über eine Leitung (36) zu einem der Eingänge eines Multiplexers (37), auf den noch näher eingegangen werden wird. Vom Ausgang des Multiplexers (37) gelangt das Signal über eine Leitung (38) zu einem Analog/Digital-Wandler (39), der das Signal in entsprechende digitale Daten umsetzt, die über eine Leitung (40) zu dem Mikroprozessor (5) gelangen.

Anhand dieser digitalen Daten ermittelt der Mikroprozessor (5) den zeitlichen Verlauf des von dem Temperatursensor (33) stammenden Signals und damit den zeitlichen Verlauf der Temperatur des venösen Blutes im rechten Ventrikel. Anhand der so gewonnenen Daten stellt der Mikroprozessor (5) das die Stimulationsfrequenz bestimmende Zeitintervall zwischen einer oberen und einer unteren Grenze, die jeweils vorgebbar sind, so ein, dass die Stimulation des Herzens (4) im Bedarfsfalle mit einer an die körperlicher Aktivität des Lebewesens angepassten Frequenz erfolgt. Ein entsprechender Algorithmus ist beispielsweise in der US-PS 4,543,954 beschrieben.

Bekanntlich müssen die Änderungen der Körpertemperatur und auch Änderungen der Temperatur des venösen Blutes nicht zwangsläufig durch eine entsprechende Änderung der körperlichen Aktivität eines Lebewesens verursacht sein. Es besteht also die Gefahr, dass das die Stimulationsfrequenz bestimmende Zeitintervall im Zusammenhang mit einer Änderung der mittels des Temperatursensors (33) gemessenen Temperatur vom Mikroprozessor (5) geändert wird, ohne dass dies im Hinblick auf die körperliche Aktivität des Lebewesens erforderlich ist.

Um dies zu verhindern, ist im Falle des erfindungsgemässen Herzschrittmachers (1) ein zweiter Temperatursensor (41) vorgesehen, der, wie dies in FIG 1 schematisch angedeutet ist, im Inneren des Gehäuses (2) des Herzschrittmachers (1) angeordnet ist. Während also der zur Messung der Temperatur des venösen Blutes dienende Temperatursensor (33) ein einer im Bereich des Körperkernes des Lebewesens vorhandenen Temperatur entsprechendes Signal liefert, entspricht das Signal des Temperatursensors (41) in Anbetracht des Umstandes, dass Herzschrittmacher zwischen Unterhautgewebe und Muskelgewebe eingesetzt werden, einer Temperatur im Bereich der Körperperipherie des Lebewesens.

Das Signal des Temperatursensors (4) gelangt über eine Leitung (42) zu einem Operationsverstärker (43). Von dessen Ausgang gelangt es über die Leitung (44) zu dem Multiplexer (37). Die Ausgangssignale der Operationsverstärker (35) und (43) gelangen zu dem invertierenden bzw. nicht invertierenden Eingangs eines Differenzverstärkers (45), der ein der Differenz der mit den Temperatursensoren (33) und (41) gemessenen Temperaturen entsprechendes Signal bildet, das über eine Leitung (46) zu einem weiteren Eingang des Multiplexers (37) gelangt. Der Multiplexer (37) wird über eine Taktleitung (47) von dem Mikroprozessor (5) derart gesteuert, dass er aufeinanderfolgend die Ausgangssignale des Operationsverstärkers (35) des Differenzverstärkers (45) und des Operationsverstärkers (43) dem Analog/Digital-Wandler (39) zuführt, der entsprechende digitale Daten über die Leitung (40) an den Mikroprozessor (5) gibt. Dieser analysiert dann neben dem zeitlichen Verlauf der mittels des Temperatursensors (33) gemessenen Temperatur auch die zeitlichen Verläufe der mittels des Temperatursensors (41) gemessenen Temperatur sowie der Differenz beider Temperaturen. Dabei ermittelt er neben dem zeitlichen Verlauf der beiden absoluten Temperaturen auch den zeitlichen Verlauf der entsprechenden Steigungen nach Betrag und Vorzeichen. Bezüglich der Differenz beider Temperaturen, die übrigens nicht notwendigerweise mittels eines Differenzverstärkers (45) bestimmt werden muss, da sie der Mikroprozessor (5) auch durch eine entsprechende Rechenoperation aus den Ausgangssignalen der Operationsverstärker (35,43) ermitteln kann, analysiert der Mikroprozessor (5) als Funktion der Zeit, ob die Differenz einen Normalwert der Differenz über- oder unterschreitet. Darüberhinaus ermittelt der Mikroprozessor (5) den Wert der Differenz der beiden Temperaturen als Funktion der Zeit und ausserdem die Steigung dieses Verlaufes nach Betrag und Vorzeichen.

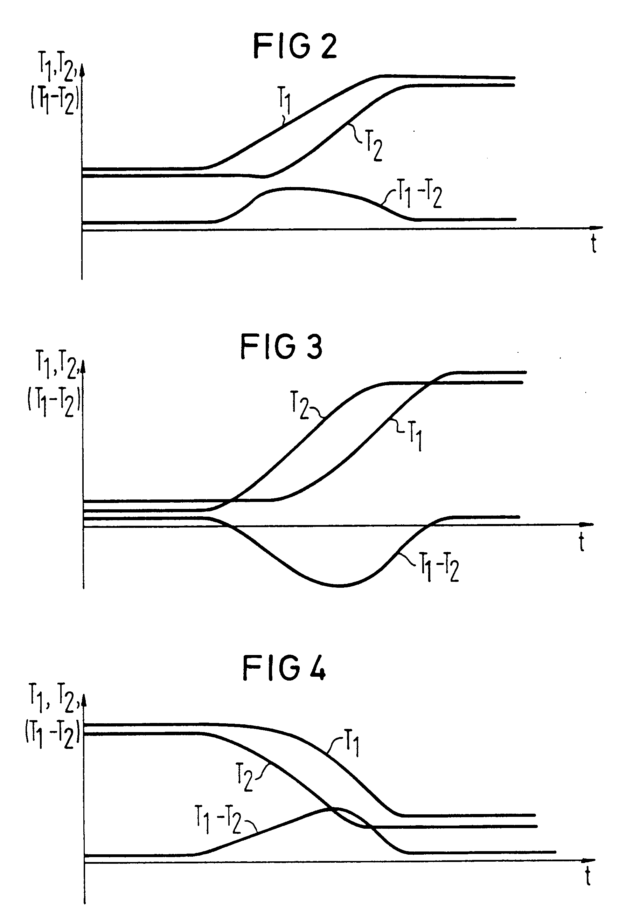

Ob eine Änderung, insbesondere eine Erhöhung, der im Bereich des Körperkernes des Lebewesens vorliegenden, mit Hilfe des Temperatursensors (33) ermittelten Temperatur auf einer Änderung, insbesondere Zunahme, der körperlichen Aktivität des Lebewesens beruht, und demzufolge eine entsprechende Anpassung der Stimulationsfrequenz bzw. des diese bestimmenden Zeitintervalles erforderlich ist, vermag der Mikroprozessor (5) bereits anhand des Vorzeichens der Steigung des zeitlichen Verlaufs einer der beiden Temperaturen, z.B. der im Bereich des Körperkernes vorliegenden Temperatur, sowie daran zu erkennen, ob die Differenz der beiden Temperaturen einen bei bezüglich der Temperaturen stationären Verhältnissen vorliegenden Normalwert über- oder unterschreitet. Dies wird anhand der Figuren 2 bis 4 deutlich. In diesen Figuren sind die Verläufe der mittels der Temperatursensoren (33) und (41) gemessenen Temperaturen über einen Zeitraum in der Grössenordnung von 15 bis 60 Minuten dargestellt. Der mit T₁ bezeichnete Temperaturverlauf entspricht der mittels des Temperatursensors (33) gemessenen Temperatur im Bereich des Körperkernes, während der mit T₂ gekennzeichnete Temperaturverlauf der mittels des Temperatursensors (41) gemessenen Temperatur im Bereich der Körperperipherie entspricht. Die dargestellten Schwankungen der Temperaturen T₁ und T₂ liegt in der Grössenordnung von 1 Grad Kelvin. In die Figuren 2 bis 4 ist ausserdem der zeitliche Verlauf der Differenz der Temperaturen T₁ minus T₂ dargestellt. Die Maximalwerte der Differenz T₁ minus T₂ liegen in der Grössenordnung von einigen Zehntelgrad Kelvin.

In der FIG 2 sind die Verhältnisse dargestellt, wie sie auftreten, wenn ausgehend von einem stationären Zustand eine Zunahme der körperlichen Aktivität des Lebewesens auftritt. In diesem Falle wird im Inneren des Körpers des Lebewesens Wärme erzeugt. Dieser Effekt wird noch dadurch verstärkt, dass erwärmtes Blut von den arbeitenden Muskeln zum Herzen des Lebewesens im Zentrum von dessen Brustraum gepumpt wird. Es tritt daher zuerst ein Anstieg der im Bereich des Körperkernes vorliegenden Temperatur T₁ auf, ohne dass sich die Temperatur T₂ im Bereich der Körperperipherie ändert. Erst nach einer gewissen Zeit erfolgt auch ein Anstieg der Temperatur T₂, bis sich nach weiter andauernder körperlicher Aktivität ein neues Gleichgewicht einstellt. Sowohl vor der Zunahme der körperlichen Aktivität als auch nach Erreichen eines neuen Gleichgewichtes besitzt die Differenz der Temperaturen T₁ minus T₂ ihren Normalwert N, der relativ gering ist. Während des zeitlich versetzten Anstieges der Temperaturen T₁ und T₂ nimmt die Temperaturdifferenz T₁ minus T₂ jedoch relativ stark zu und überschreitet dabei den Normalwert N.

Es wird somit deutlich, dass bei Zunahme der körperlichen Aktivität das Vorzeichen der Steigung des zeitlichen Verlaufs beider Temperaturen T₁ und T₂ positiv ist und dass während der Zunahme der körperlichen Aktivität die Temperaturdifferenz T₁ minus T₂ ihren Normalwert N überschreitet.

In FIG 3 sind die Verhältnisse dargestellt, die auftreten, wenn die Temperatur des das Lebewesen umgebenden Mediums steigt. Dies ist z.B. der Fall, wenn sich das Lebewesen von einem kalten Raum in einen wärmeren Raum begibt, ein heisses Bad nimmt oder warme Kleidung anlegt. In diesem Falle tritt infolge der von aussen erfolgenden Wärmezufuhr zunächst ein Anstieg der Temperatur T₂ im Bereich der Körperperipherie auf, der später auch ein Anstieg der Temperatur T₁ im Bereich des Körperkernes folgt. Wie bei einer Zunahme der körperlichen Aktivität tritt also auch im Falle einer Zunahme der Temperatur des das Lebewesen umgebenden Mediums ein Anstieg der Temperaturen T₁ und T₂ auf, was bedeutet, dass die Steigungen der zeitlichen Verläufe der Temperaturen T₁ und T₂ jeweils ebenfalls ein positives Vorzeichen besitzen. Im Gegensatz zu einer Zunahme der körperlichen Aktivität unterschreitet jedoch im Falle einer Zunahme der Temperatur des das Lebewesen umgebenden Mediums die Temperaturdifferenz T₁ minus T₂ den Normalwert N und kann sogar negative Werte annehmen.

Das sich nach einiger Zeit einstellende neue Temperaturgleichgewicht hängt von den jeweiligen Verhältnissen ab. So ist es im Falle eines heissen Bades möglich, als Kühlungsmechanismus steht dem Körper des Lebewesens hier im wesentlichen nur das Atmungssystem zur Verfügung, dass auch nach einer längeren Zeitdauer die Differenz der Temperaturen T₁ minus T₂ den Normalwert N noch unterschreitet bzw. einen negativen Wert besitzt.

Die in FIG 4 dargestellten Verhältnisse beziehen sich auf eine rasche Abkühlung des Körpers des Lebewesens, wie sie z.B. im Falle eines kalten Bades auftritt. In diesem Falle fällt zuerst die Temperatur T₂ im Bereich der Körperperipherie des Lebewesens ab, bevor auch eine Abnahme der Temperatur T₁ im Bereich des Körperkernes auftritt. Die dabei auftretende Differenz der Temperaturen T₁ minus T₂ überschreitet wie im Falle einer Zunahme der körperlichen Aktivität des Lebewesens den Normalwert N. Im Gegensatz zu der Zunahme der körperlichen Aktivität bzw. einem Anstieg der Temperatur des des Lebewesen umgebenden Mediums besitzen die Steigungen der zeitlichen Verläufe der Temperaturen T₁ und T₂ jedoch negative Vorzeichen.

Es wird somit deutlich, dass der Mikroprozessor (5) bereits unter Berücksichtigung des zeitlichen Verlaufs einer der Temperaturen T₁ oder T₂ dem Vorzeichen seiner Steigung nach und des zeitlichen Verlaufs der Differenz der Temperaturen T₁ minus T₂ hinsichtlich des Über- oder Unterschreitens des Normalwertes N in der Lage ist, das die Stimulationsfrequenz bestimmende Zeitintervall im Hinblick auf Veränderungen der Temperatur T₁ nur dann zu verstellen, wenn die Änderung der Temperatur T₁ tatsächlich durch eine Änderung der körperlichen Aktivität des Lebewesens verursacht ist. Damit ist gewährleistet, dass eine Anpassung, insbesondere eine Erhöhung, der Stimulationsfrequenz nur erfolgt, wenn tatsächlich eine entsprechende Änderung der körperlichen Aktivität des Lebewesens vorliegt.

Wenn der Mikroprozessor (5) bei der Einstellung des die Stimulationsfrequenz bestimmenden Zeitintervalls ausserdem den Betrag der Steigung der im Bereich des Körperkernes vorliegenden Temperatur T₁ berücksichtigt, sind für den Fall, dass die jeweilige Änderung der Temperatur T₁ auf eine Änderung der körperlichen Aktivität des Lebewesens zurückzuführen ist, unterschiedliche Reaktionsweisen realisierbar, je nachdem ob der Betrag der Steigung des zeitlichen Verlaufs der Temperatur T₁ auf eine plötzliche oder sehr langsame Änderung der körperlichen Aktivität des Lebewesens hindeutet. Aus ähnlichen Gründen berücksichtigt der Mikroprozessor (5) die Vorzeichen der Steigungen der zeitlichen Verläufe beider Temperatur T₁ und T₂ sowie den Betrag der Steigung des zeitlichen Verlaufes der im Bereich der Körperperipherie des Lebewesens vorliegenden Temperatur T₂ . Ergibt ein von dem Mikroprozessor (5) durchgeführter Vergleich der zeitlichen Verläufe der Temperatur T₁ und T₂ im Zusammenhang mit einer Änderung der körperlichen Aktivität des Lebewesens bei gleichem Vorzeichen der Steigung der zeitlichen Verläufe der Temperaturen T₁ und T₂ erhebliche Unterschiede der Steigungen dem Betrag nach oder gar unterschiedliche Vorzeichen der Steigungen, ist dies ein Anzeichen dafür, dass gleichzeitig mit der Änderung der körperlichen Aktivität des Lebewesens eine erhebliche Änderung der Temperatur des das Lebewesen umgebenden Mediums stattfindet, wie dies z.B. der Fall ist, wenn das Lebewesen in kühlem Wasser zu schwimmen beginnt. Auch derartige Informationen berücksichtigt der Mikroprozessor (5) bei der Einstellung des die Stimulationsfrequenz bestimmenden Zeitintervalles. Weitere Informationen bezüglich etwaiger Temperaturänderungen des das Lebewesen umgebenden Mediums gewinnt der Mikroprozessor (5) durch die Analyse des zeitlichen Verlaufs des Wertes der Differenz der Temperaturen T₁ minus T₂. So bedeutet ein negativer Wert der Differenz T₁ minus T₂ z.B. grundsätzlich, dass die Temperatur des das Lebewesen umgebenden Mediums oberhalb der Körpertemperatur des Lebewesens liegt. Auch diesen Sachverhalt berücksichtigt der Mikroprozessor (5) bei der Einstellung des die Stimulationsfrequenz bestimmenden Zeitintervalles.

Wie aus der FIG 1 ersichtlich ist, steht der Mikroprozessor (5) über eine Leitung (48) mit einem schematisch angedeuteten Telemetrieschaltkreis (49), an den eine Sende/Empfangs-Spule (50) angeschlossen ist, in Verbindung. Mittels des Telemetrieschaltkreises (49) ist ein bidirektionaler Datenverkehr mit einem externen Gerät, einem sogenannten Programmer, möglich. Auf diese Weise ist es möglich, die in dem RAM (7) gespeicherten Variablen zur Überprüfung abzurufen bzw. zu verändern.

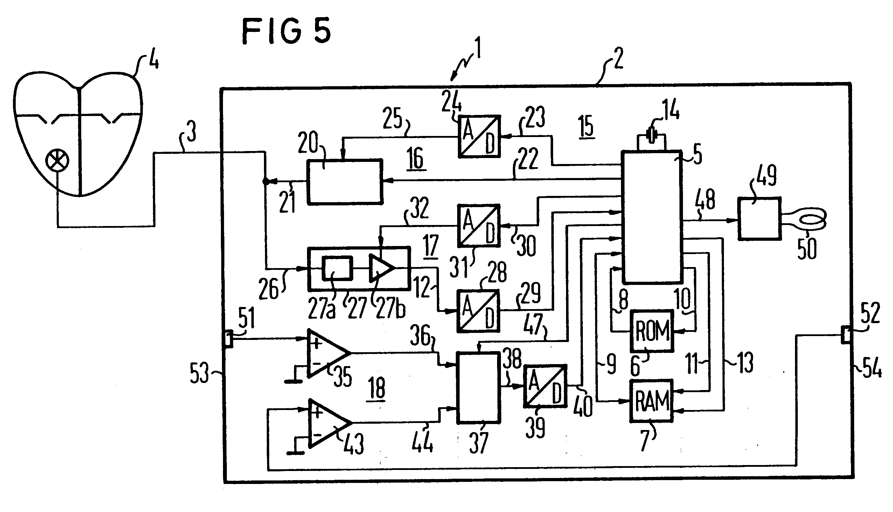

Der in FIG 5 dargestellte Herzschrittmacher (1) entspricht im wesentlichen dem zuvor beschriebenen Herzschrittmacher, weshalb gleiche Komponenten mit gleichen Bezugszeichen versehen sind.

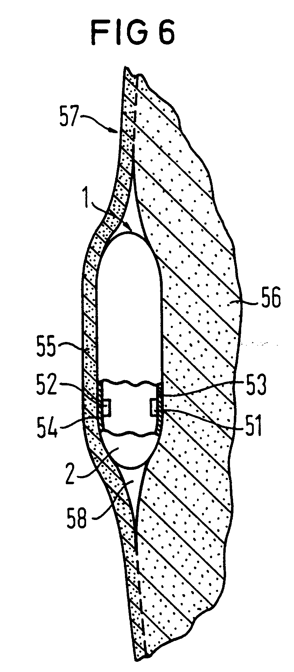

Ein wesentlicher Unterschied zu dem zuvor beschriebenen Herzschrittmacher besteht darin, dass bei dem Herzschrittmacher (1) gem. FIG 5 beide Temperatursensoren (51,52) im Inneren des Gehäuses (2) angeordnet sind. Wie in FIG 5 schematisch angedeutet ist, sind die Temperatursensoren (51,52) jeweils wärmeleitend mit unterschiedlichen Abschnitten der Innenwand des Gehäuses (2) verbunden. Dies wird aus der Darstellung der FIG 6, in der von dem Herzschrittmacher (1) gem. FIG 5 nur das Gehäuse (2) und die Temperatursensoren (51,52) dargestellt sind, nochmals deutlicher, da hier erkennbar ist, dass die Temperatursensoren (51,52) an einander gegenüberliegenden Wandabschnitten (53,54) des Gehäuses (2) angebracht sind. Wie aus der FIG 6 weiter deutlich wird, ist der Herzschrittmacher (1) derart in eine zwischen dem Unterhautgewebe (55) und dem Muskelgewebe (56) des Körpers (57) des Lebewesens befindliche Tasche (58) implantiert, dass der Wandabschnitt (53) mit dem Temperatursensor (51) dem Muskelgewebe (56) und damit dem Körperkern des Lebewesens zugewandt ist. Der Wandabschnitt (54) mit dem Temperatursensor (52) ist dem Unterhautgewebe (55) und damit der Körperperipherie des Lebewesens zugewandt. Es wird somit deutlich, dass die mittels des Temperatursensors (51) gemessene Temperatur T₁ der Temperatur im Bereich des Körperkernes und die mittels des Temperatursensors (52) gemessene Temperatur T₂ der Temperatur im Bereich der Körperperipherie des Lebewesens entspricht. Sofern das Gehäuse (2) des Herzschrittmachers (1) aus einem Werkstoff mit einer geringen Wärmeleitfähigkeit, z.B. Titan, gebildet ist, ist die thermische Koppelung der Temperatursensoren (51) und (52) ausreichend gering, um störende Einflüsse zu vermeiden.

Ein weiterer Unterschied des Herzschrittmachers (1) gem. FIG 5 zu dem zuvor beschriebenen besteht darin, dass im Kanal (18) der Differenzverstärker (45) fehlt. Wie erwähnt besteht zwar grundsätzlich die Möglichkeit, dass der Mikroprozessor (5) die Differenz der Temperaturen T₁ und T₂ rechnerisch ermittelt, diese wird jedoch im Falle des Herzschrittmachers (1) gem. FIG 5 bei der Einstellung des der Stimulationsfrequenz entsprechenden Zeitintervalls primär nicht berücksichtigt. Statt dessen analysiert der Mikroprozessor (5) die zeitlichen Verläufe der Temperaturen T₁ und T₂ daraufhin, in welcher zeitlichen Reihenfolge Änderungen der zeitlichen Verläufe beider Temperaturen T₁ und T₂ auftreten.

Wie im Zusammenhang mit der Erläuterung der FIG 2 bis 4 deutlich wird, tritt im Falle einer Änderung, insbesondere eines Anstiegs, der körperlichen Aktivität des den Herzschrittmacher (1) tragenden Lebewesens zunächst eine Änderung der im Bereich des Körperkernes des Lebewesens vorliegenden Temperatur T₁ auf, der später eine entsprechende Änderung der im Bereich der Körperperipherie vorliegenden Temperatur T₂ folgt. Treten dagegen Änderungen der Körpertemperatur des Lebewesens auf, die auf äussere Ursachen zurückgehen, z.B. warmes oder kaltes Bad, tritt zuerst eine Änderung der im Bereich der Körperperipherie vorliegenden Temperatur T₂ auf, der später eine entsprechende Änderung der im Bereich des Körperkernes vorliegenden Temperatur T₁ folgt.

Der Mikroprozessor (5) ist also in der Lage, anhand der zeitlichen Reihenfolge, in der Änderungen der Temperaturen T₁ und T₂ auftreten zu erkennen, ob diese durch eine Änderung körperlichen Aktivität des Lebewesens verursacht sind. Nur wenn dies der Fall ist, stellt der Mikroprozessor (5) das die Stimulationsfrequenz bestimmende Zeitintervall unter Heranziehung der mittels des Temperatursensors (51) gemessenen absoluten Temperatur im Bereich des Körperkernes als Mass für die Intensität der körperlichen Aktivität des Lebewesens ein. Dabei können weitere aus den zeitlichen Verläufen der Temperaturen T₁ und T₂ abgeleitete Parameter einschliesslich der Differenz der Temperaturen T₁ minus T₂ in der im Zusammenhang mit dem Ausführungsbeispiel gemäss FIG 1 geschilderten Weise Berücksichtigung finden.

Bei den Temperatursensoren (33 und 41 bzw. 51 und 52) kann es sich beispielsweise um Thermistoren oder temperaturempfindliche Halbleiterdioden handeln.

Der Mikroprozessor (5), der Nur-Lese-Speicher (ROM) (6) und der Schreib-Lese-Speicher (RAM) (7) können im Falle beider Ausführungsbeispiele zu einem Ein-Chip-Computer zusammengefasst sein.

Wesentliche Funktionen der Herzschrittmacher gemäss den beschriebenen Ausführungsbeispielen sind durch einen geeignet programmierten Mikroprozessor (5) gesteuert. Die entsprechenden Funktionen können jedoch ohne weiteres auch durch eine herkömmlich aufgebaute Steuerlogik realisiert werden.

Obwohl die Erfindung ausschliesslich anhand von Herzschrittmachern erläutert wurde, kann sie auch bei anderen medizinischen Geräten Verwendung finden, bei denen eine Einstellung der Stimulationsintensität in Abhängigkeit von der körperlichen Aktivität des das Gerät tragenden Lebewesens erfolgen soll.

## Patentansprüche

1. In den Körper (57) eines Lebewesens implantierbares medizinisches Gerät, welches Mittel (3,5,20) zur Stimulation eines physiologischen Vorganges des Lebewesens mit einstellbarer Stimulationsintensität und selbsttätige Stellmittel (5,24,) zur Anpassung der Stimulationsintensität an die körperliche Aktivität des Lebewesens aufweist, die die Stimulationsintensität anhand der Signale zweier Temperatursensoren (51,52) einstellen, von denen das eine der Temperatur (T₁) im Bereich des Körperkerns und das andere der Temperatur (T₂) im Bereich der Körperperipherie des Lebewesens entspricht, **dadurch gekennzeichnet,** dass die Stellmittel (5,24) die Stimulationsintensität unter Ermittlung der zeitlichen Reihenfolge einstellen, in der Änderungen der zeitlichen Verläufe beider Temperaturen (T₁, T₂) auftreten.

2. Gerät nach Anspruch 1, **dadurch gekennzeichnet,** dass die Stellmittel (5,24) die Stimulationsintensität ausserdem unter Berücksichtigung von wenigstens dem Vorzeichen der Steigung des zeitlichen Verlaufs zumindest einer der Temperaturen (T₁,T₂) einstellen.

3. Gerät nach Anspruch 1, **dadurch gekennzeichnet,** dass die Stellmittel (5,24) die Stimulationsintensität unter Berücksichtigung des zeitlichen Verlaufs zumindest einer der Temperaturen (T₁,T₂) Wenigstens dem Vorzeichen der Steigung nach und unter Berücksichtigung des zeitlichen Verlaufs der Differenz der beiden Temperaturen (T₁,T₂) wenigstens hinsichtlich des Über- oder Unterschreitens eines Normalwertes (N) der Differenz einstellen.

4. Gerät nach einem der Ansprüche 1 bis 3, **dadurch gekennzeichnet,** dass die Stellmittel (5,24) die Stimulationsintensität ausserdem unter Berücksichtigung des Betrages der Steigung des zeitlichen Verlaufs wenigstens einer der Temperaturen (T₁,T₂) einstellen.

5. Gerät nach einem der Ansprüche 1 bis 4, **dadurch gekennzeichnet,** dass die Stellmittel (5,24) die Stimulationsintensität unter Berücksichtigung wenigstens der Vorzeichen der Steigungen der zeitlichen Verläufe beider Temperaturen (T₁,T₂) einstellen.

6. Gerät nach einem der Ansprüche 1 bis 5, **dadurch gekennzeichnet,** dass das Gerät als Herzschrittmacher (1) ausgeführt ist, wobei die Mittel (3,5,20) zur Stimulation eines physiologischen Vorganges die Herztätigkeit des Lebewesens stimulieren und die Stellmittel (5,24) zum Anpassen der Stimulationsintensität die Stimulationsfrequenz, mit der die Mittel (3,5,20) zur Stimulation das Herz (4) des Lebewesens im Bedarfsfalle stimulieren, an die körperliche Aktivität des Lebewesens anpassen.

7. Gerät nach einem der Ansprüche 1 bis 6, welches ein Gehäuse (2) mit zwei einander gegenüberliegenden Wandabschnitten (53,54) aufweist, **dadurch gekennzeichnet,** dass jedem der beiden Wandabschnitte (53,54) einer der Temperatursensoren (51,52) zugeordnet und jeweils im Inneren des Gehäuses (2) wärmeleitend an dem entsprechenden Wandabschnitt (53,54) angebracht ist.

8. Gerät nach Anspruch 6, welches von einem Gehäuse (2) umgeben ist und eine mit ihrem einen Ende in das rechte Ventrikel des Herzens (4) implantierbare Elektrode (3) aufweist, die mit einem im Bereich des einen Endes der Elektrode (3) befindlichen Temperatursensor (33) zur Messung der in dem rechten Ventrikel vorliegenden Bluttemperatur (T₁) versehen ist, **dadurch gekennzeichnet,** dass sich im Inneren des Gehäuses (2) der andere Temperatursensor (41) befindet.

9. Verfahren zur Anpassung der einstellbaren Stimulationsintensität eines medizinischen Gerätes zur Stimulation eines physiologischen Vorganges eines Lebewesens an die körperliche Aktivität des Lebewesens, wobei die Stimulationsintensität anhand der Temperatur (T₁) im Bereich des Körperkernes und der Temperatur (T₂) im Bereich der Körperperipherie des Lebewesens eingestellt wird, **dadurch gekennzeichnet,** dass die Stimulationsintensität unter Ermittlung der zeitlichen Reihenfolge, in der Änderungen der zeitlichen Verläufe beider Temperaturen (T₁,T₂) auftreten, eingestellt wird.

10. Verfahren nach Anspruch 9, **dadurch gekennzeichnet,** dass die Stimulationsintensität ausserdem unter Berücksichtigung von wenigstens dem Vorzeichen der Steigung des zeitlichen Verlaufs zumindest einer der Temperaturen (T₁, T₂) eingestellt wird.

11. Verfahren nach Anspruch 9, **dadurch gekennzeichnet,** dass die Stimulationsintensität unter Berücksichtigung des zeitlichen Verlaufs zumindest einer der Temperaturen (T₁,T₂) wenigstens dem Vorzeichen der Steigung nach und unter Berücksichtigung des zeitlichen Verlaufs der Differenz der beiden Temperaturen (T₁,T₂) der beiden Temperaturen wenigstens hinsichtlich des Über- oder Unterschreitens eines Normalwertes (N) der Differenz eingestellt wird.

## Claims

1. Medical appliance implantable in the body (57) of an individual, which has means (3, 5, 20) for stimulating a physiological process of the individual with adjustable intensity of stimulation and automatic control means (5, 24) for matching the intensity of stimulation to the bodily activity of the individual, which adjust the intensity of stimulation with reference to the signals of two temperature sensors (51, 52), of which one corresponds to the temperature (T₁) in the region of the body core and the other corresponds to the temperature (T₂) in the region of the body periphery of the individual, characterized in that the control means (5, 24) adjust the intensity of stimulation while determining the secular sequence in which changes occur in the secular variations of the two temperatures (T₁, T₂).

2. Appliance according to Claim 1, characterized in that, furthermore, the control means (5, 24) adjust the intensity of stimulation taking account of at least the sign of the gradient of the secular variation of at least one of the temperatures (T₁, T₂).

3. Appliance according to Claim 1, characterized in that the control means (5, 24) adjust the intensity of stimulation taking account of the secular variation of at least one of the temperatures (T₁, T₂) at least in accordance with the sign of the gradient and taking account of the secular variation in the difference of the two temperatures (T₁, T₂) at least with regard to the overshooting or undershooting of a standard value (N) of the difference.

4. Appliance according to one of Claims 1 to 3, characterized in that, furthermore, the control means (5, 24) adjust the intensity of stimulation taking account of the magnitude of the gradient of the secular variation of at least one of the temperatures (T₁, T₂).

5. Appliance according to one of Claims 1 to 4, characterized in that the control means (5, 24) adjust the intensity of stimulation taking account of at least the sign of the gradients of the secular variations of the two temperatures (T₁, T₂).

6. Appliance according to one of Claims 1 to 5, characterized in that the appliance is embodied as a cardiac pacemaker (1), the means (3, 5, 20) for stimulating a physiological process stimulating the cardiac activity of the individual, and the control means (5, 24) for matching the intensity of stimulation matching the frequency of stimulation with which the means (3, 5, 20) of stimulation stimulate the heart (4) of the individual in case of need to the bodily activity of the individual.

7. Appliance according to one of Claims 1 to 6, which has a housing (2) with two mutually opposite wall sections (53, 54), characterized in that each of the two wall sections (53, 54) is assigned one of the temperatures sensors (51, 52), which is mounted in a thermally conductive fashion on the appropriate wall section (53, 54) on the inside of the housing (2) in each case.

8. Appliance according to Claim 6, which is surrounded by a housing (2) and has an electrode (3) implantable with its end in the right ventricle of the heart (4), which electrode is provided with a temperature sensor (33), situated in the region of one end of the electrode (3), for measuring the blood temperature (T₁) occurring in the right ventricle, characterized in that the other temperature sensor (41) is situated in the inside of the housing (2).

9. Method of matching the adjustable intensity of stimulation of a medical appliance for stimulating a physiological process of an individual to the bodily activity of the individual, the intensity of stimulation being adjusted with reference to the temperature (T₁) in the region of the body core and the temperature (T₂) in the region of the body periphery of the individual, characterized in that the intensity of stimulation is adjusted while determining the secular sequence in which changes occur in the secular variations of the two temperatures (T₁, T₂)

10. Method according to Claim 9, characterized in that, furthermore, the intensity of stimulation is adjusted taking account of at least the sign of the gradient of the secular variation of at least one of the temperatures (T₁, T₂).

11. Method according to Claim 9, characterized in that the intensity of stimulation is adjusted taking account of the secular variation of at least one of the temperatures (T₁, T₂) at least in accordance with the sign of the gradient and taking account of the secular variation in the difference of the two temperatures (T₁, T₂) at least with regard to the overshooting or undershooting of a standard value (N) of the difference.

## Revendications

1. Appareil médical implantable dans le corps (57) d'un être vivant et qui comporte des moyens (3, 5, 20) pour stimuler un processus physiologique de l'être vivant avec une intensité de stimulation réglable, et des moyens de réglage automatique (5, 24) pour adapter l'intensité de stimulation à l'activité corporelle de l'être vivant et qui règlent l'intensité de stimulation sur la base des signaux de deux capteurs de température (51, 52), dont l'un correspond à la température (T₁) à l'intérieur du corps et dont l'autre correspond à la température (T₂) dans la zone de la périphérie du corps de l'être vivant, caractérisé par le fait que les moyens de réglage (5, 24) règlent l'intensité de stimulation moyennant la détermination de la séquence temporelle, avec laquelle apparaissent des modifications des variations dans le temps des deux températures (T₁, T₂).

2. Appareil suivant la revendication 1, caractérisé par le fait que les moyens de réglage (5, 24) règlent en outre l'intensité de stimulation en tenant compte au moins du signe de la pente de la courbe de variation dans le temps d'au moins l'une des températures (T₁, T₂).

3. Appareil suivant la revendication 1, caractérisé par le fait que les moyens de réglage (5, 24) règlent l'intensité de stimulation en tenant compte de la variation dans le temps d'au moins l'une des températures (T₁, T₂), au moins en fonction du signe de la pente et en tenant compte de la variation dans le temps de la différence des deux températures (T₁, T₂) au moins en rapport avec le dépassement par valeurs supérieures ou par valeurs inférieures d'une valeur normale (N) de la différence.

4. Appareil suivant l'une des revendications 1 à 3, caractérisé par le fait que les moyens de réglage (5, 26) règlent l'intensité de stimulation en tenant compte, en outre, de la valeur absolue de la pente de la variation dans le temps d'au moins l'une des températures (T₁, T₂).

5. Appareil suivant l'une des revendications 1 à 4, caractérisé par le fait que les moyens de réglage (5, 24) règlent l'intensité de stimulation en tenant compte au moins des signes des pentes des variations dans le temps des deux températures (T₁, T₂).

6. Appareil suivant l'une des revendications 1 à 5, caractérisé par le fait que l'appareil est réalisé sous la forme d'un stimulateur cardiaque (1), les moyens (3, 5, 20) servant à stimuler un processus physiologique stimulant l'activité du coeur de l'être vivant, tandis que les moyens de réglage (5, 24) servant à adapter l'intensité de stimulation adaptent l'intensité de stimulation, avec laquelle les moyens (3, 5, 20) de stimulation réalisent en cas de besoin la stimulation du coeur (4) de l'être vivant, à l'activité corporelle de ce dernier.

7. Appareil suivant l'une des revendications 1 à 6, qui comporte un boîtier (2) possédant deux sections de paroi réciproquement opposées (53, 54), caractérisé par le fait que l'un des capteurs de température (51, 52) est associé à chacune des deux sections de paroi (51, 54) et est monté respectivement à l'intérieur du boîtier (2), de manière à conduire la chaleur, sur la section de paroi correspondante (53, 54).

8. Appareil suivant la revendication 6, qui est entouré par un boîtier (2) et possède une électrode (3) implantable, par l'une de ses extrémités, dans le ventricule droit du coeur (4) et qui est pourvu d'un capteur de température (33), qui est situé au voisinage de ladite extrémité de l'électrode (3) pour la mesure de la température (T₁) du sang, qui règne dans le ventricule droit, caractérisé par le fait que l'autre capteur de température (41) est situé à l'intérieur du boîtier (2).

9. Procédé pour adaptater l'intensité de stimulation réglable d'un appareil médical pour la stimulation d'un processus physiologique d'un être vivant à l'activité corporelle de ce dernier, l'intensité de stimulation étant réglée en fonction de la température (T₁) à l'intérieur du corps, tandis que la température (T₂) est réglée dans la zone de la périphérie du corps de l'être vivant, caractérisé par le fait que l'intensité de stimulation est réglée au moyen de la détermination de la séquence temporelle avec laquelle apparaissent des modifications des variations dans le temps des deux températures (T₁, T₂).

10. Procédé suivant la revendication 9, caractérisé par le fait que l'intensité de stimulation est, en outre, réglée en tenant compte au moins du signe de la pente de la variation dans le temps d'au moins l'une des températures (T₁, T₂).

11. Procédé suivant la revendication 9, caractérisé par le fait que l'intensité de stimulation est réglée en tenant compte de la variation dans le temps de l'une des températures (T₁, T₂), au moins en fonction du signe de la pente et en tenant compte de la variation, dans le temps, de la différence des deux températures (T₁, T₂), au moins en rapport avec le dépassement par valeurs supérieures ou par valeurs inférieures d'une valeur normale (N) de la différence.
